# EUROPEAN PATENT APPLICATION

(11) **EP 4 215 168 A1**
(43) Date of publication of application: **26.07.2023**
(21) Application number: 23151676.6
(22) Date of filing: 16.01.2023
(51) Int. Cl.: A61F 7/00, A61F 7/02

(54) **COLD/HOT COMPRESS STRIP DEVICE WITH QUICK REPLACEABLE COMPRESS STRIP AND EFFICIENT COLD/HOT SWITCH**

(30) Priority: 24.01.2022 TW 111102995
(71) Applicant: Hwang, Chin-Hwa, Taipei City (TW)
(72) Inventor: Hwang, Chin-Hwa, Taipei City (TW)
(74) Representative: Zeitler Volpert Kandlbinder Patentanwälte Partnerschaft mbB

(57) **Abstract**

A cold/hot compress strip device with a quick replaceable compress strip and an efficient cold/hot switch includes a host machine and a flexible compress strip fixable to an affected part on a human body surface, and the host machine can pump the cooled cold water from its container into the compress strip and circulate the cold water in the compress strip for cold compress, or can quickly discharge the cold water in the compress strip, and drive a heating film on the compress strip to generate heat for hot compress, so as to achieve an efficient cold/hot switch, and the compress strip can be replaced quickly as needed. The application of the cold/hot compress strip device is very simple and practical.

## Description

### FIELD OF THE INVENTION

The present invention relates to a cold/hot compress strip device, and more particularly to the cold/hot compress strip device that separates a cooling source and a heating source, which not only can provide an efficient cold/hot switch to save energy, but also can save the manufacturing cost by using conventional thermal conduction and heat dissipation components, and can quickly replace the compress strip according to a user requirement for different affected parts.

### BACKGROUND OF THE INVENTION

Most of the current cold/hot compress strip devices use an electrothermal cooling chip as a temperature generating source, and the principle is that after an N-type semiconductor and P-type semiconductor on the electrothermal cooling chip are electrically conducted, current flows from the N-type semiconductor to the P-type semiconductor to absorb heat energy to form a cooling end. If the current direction is changed, the current flowing from the P-type semiconductor to the N-type semiconductor can release heat energy to form a heating end, so that the electrothermal cooling chip can achieve the cooling and heating functions.

The cold/hot compress strip device usually use cold water or hot water as a cold/hot conduction medium of the electrothermal cooling chip, so that the cold water and hot water can circulate in a preset pipeline to achieve the cold compress or hot compress effect. For example, a massage device as disclosed in R.O.C. Pat. No. 1614012 mainly includes a water pump and an electrothermal cooling chip installed in a water tank, and a plurality of tubes communicating with a water bag, such that the electrothermal cooling chip can control the circulating water in the water tank to be heated or refrigerated to a predetermined temperature, and the water pump pumps the circulating water from the water tank into the water bag through the plurality of tubes, and then recirculates the water back into the water tank, so that the circulating water in the water bag can keep a constant temperature, and achieve the cold compress or hot compress effect when the water bag is placed against a user's skin.

In the aforementioned prior art, both cold and hot compress functions are achieved by using the same electrothermal cooling chip, same circulation pipeline and same water tank, so that when the cold compress is switched to the hot compress, it is necessary to heat the cold water/ice water to achieve the hot compress effect. Similarly, when the hot compress is switched to the cold compress, it is necessary to cool the warm or hot water to the cold water/ice water to achieve the cold compress effect. Obviously the heating and cooling processes are slow and inefficient, and consume much power and waste energy.

In view of the aforementioned drawbacks of the conventional cold/hot compress device using the same refrigerating/heating source and circulation pipeline, having a low efficiency of switching between cold compress and hot compress, and wasting energy, it is a primary objective of the present invention to overcome the drawbacks of the prior art by providing a cold/hot compress strip device of the present invention, while making an effective use of limited costs.

### SUMMARY OF THE INVENTION

Specifically, the present invention relates to a cold/hot compress strip device with a quick replaceable compress strip and an efficient cold/hot switch, and the cold/hot compress strip device includes a host machine, and a flexible compress strip detachably connected to the host machine and attachable to a human body surface, wherein:

The host machine includes a set of upper and lower casings vertically engaged with each other, and the lower casing has a partition plate disposed therein for dividing the lower casing into first and second accommodation spaces, and the first accommodation space contains a container for containing cold water, and the top of the container has a cover for closing the container, and the cover has an opening formed thereon; and the second accommodation space contains a water pump, a water valve, and a control unit, for pumping and circulating the cold water.

The upper casing has a cooling chip installed at a position above the opening of the cover and the cooling chip has a cooling surface and a heat dissipation surface, and the cooling surface is stacked with a plurality of temperature conduction fins fixed to an inner periphery of the opening and extending into the container, and the heat dissipation surface of the cooling chip is stacked with a plurality of heat dissipation fins fixed to the temperature conduction fin and extending in an opposite direction away from the container.

The compress strip is formed by stacking two soft substrates with each other, and a water path between the two substrates is provided for circulating the cold water, and a plurality of tubes disposed between the water path and the water pump of the host machine and installed between the upper and lower casings are communicated with each other, and an outer surface of one of the substrates has a heating film separated from the water path.

The control unit of the host machine is electrically connected to the water pump, the water valve and the cooling chip, and electrically connected to the heating film of the compress strip through a power cable for controlling the ON/OFF of the water valve and the tubes, so that the water pump can pump the cold water contained in the container and cooled by the cooling chip from the plurality of tubes to the water path in the compress strip for circulation to perform a cold compress; or empty the cold water contained in the compress strip back into the container, and control the heating film to generate heat to perform a hot compress, so as to achieve an efficient cold/hot switch effect

By the aforementioned structure of the present invention, the cooling source and the heating source are separated. After the compress strip is attached and fixed onto an affected part on a human body surface, the cooling chip can be used to lower the temperature of the cold water contained in the container and circulate the cold water to the compress strip in order to perform a cold compress. For hot compress, the cold water contained in the compress strip is emptied first, and then heated by the heating film to perform a hot compress. In order to avoid the cold water from being heated in the compress strip, the cold water needs not to be cooled and heated repeatedly as in the prior art, and the invention can achieve the efficient cold/hot switch effect of the compress strip in an economic and energy-saving manner.

In addition, the internal component configuration of the host machine of the present invention is reasonable and compact, and the conventional cooling chip, temperature conduction fin and heat dissipation fin can be used to achieve the functions of conducing cold temperature and dissipating heat as well as the effect of saving the manufacturing cost effectively.

The technical characteristics of the present invention will become apparent with the detailed description of preferred embodiments accompanied with the illustration of related drawings.

### BRIEF DESCRIPTION OF THE DRAWINGS

FIG. 1 is a perspective view of the present invention;
FIG. 2 is an exploded view of a host machine of the present invention;
FIG. 3 is a cross-sectional view of a host machine of the present invention, showing the status of circulating cold water;
FIG. 4 is a cross-sectional view of a host machine of the present invention, showing the status of discharging cold water and pumping it back into a container;
FIG. 5 is an exploded view of a compress strip of the present invention; and
FIG. 6 is a schematic view of a fan and heat dissipation fins of the present invention.

### DESCRIPTION OF THE PREFERRED EMBODIMENTS

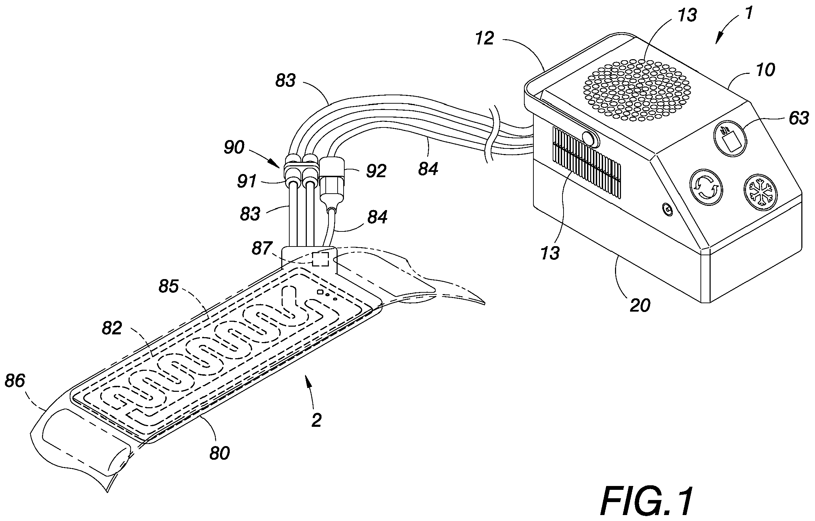

With reference to FIG. 1 for a cold/hot compress strip device with a quick replaceable compress strip and an efficient cold/hot switch in accordance with the present invention, the cold/hot compress strip device includes a host machine 1, and a compress strip 2 detachably connected to the host machine 1, and the compress strip 2 is a flexible sheet that can be adhered to an affected part on a human body surface.

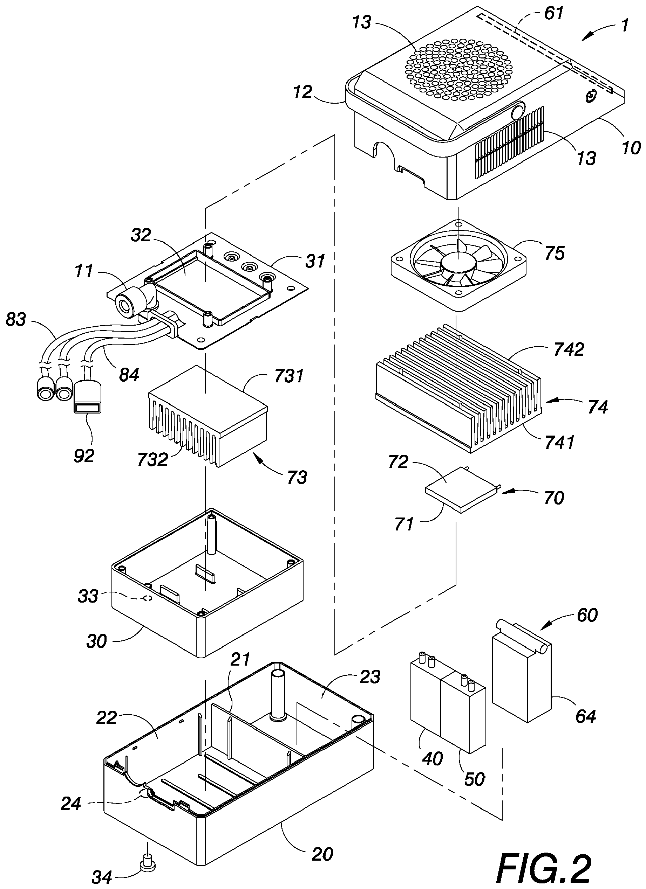

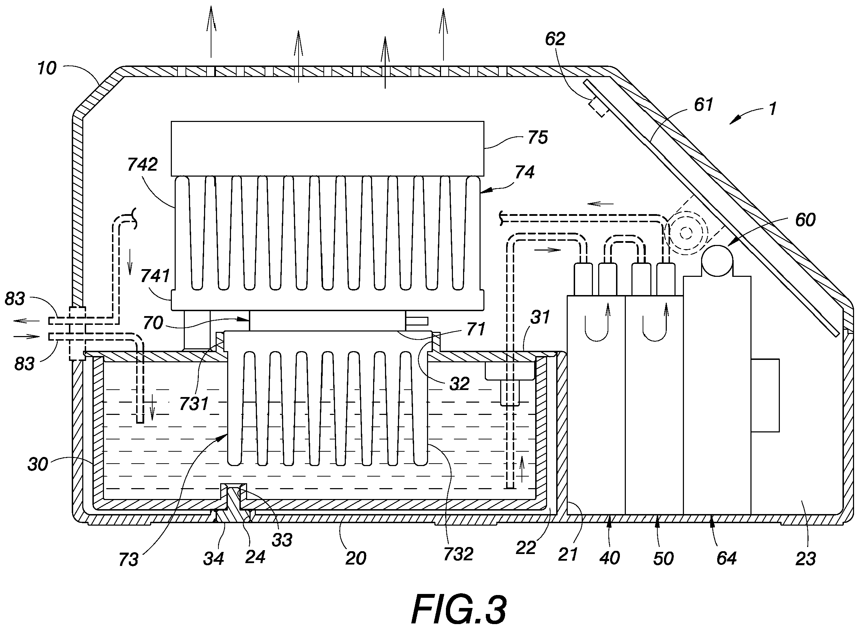

In FIGS. 2 and 3, the host machine 1 includes a set of upper and lower casings 10, 20 vertically engaged with each other, and the lower casing 20 includes a partition plate 21 installed therein for dividing the lower casing 20 into first and second accommodation spaces 22, 23, and the first accommodation space 22 includes a container 30 for containing cold water, and a cover 31 installed to the top of the cover 31 for sealing the container 30, and the cover 31 has an opening 32. The second accommodation space 23 contains a water pump 40, a water valve 50, and a control unit 60 for pumping and circulating cold water. In an embodiment, the control unit 60 can also be installed in the upper casing 10.

The upper casing 10 has a cooling chip 70 installed at a position above the opening 32 of the cover 31, and the cooling chip 70 has a cooling surface 71 and a heat dissipation surface 72, and the cooling surface 71 is stack with a plurality of temperature conduction fins 73, and the heat dissipation surface 72 is stacked with a plurality of heat dissipation fins 74, and conventional fins can be used as the temperature conduction fins 73 and the heat dissipation fins 74 in order to save the manufacturing cost.

In an embodiment, the temperature conduction fins 73 have a first heat absorption plate 731 stacked on the cooling surface 71 and a plurality of first fins 732 extending from the first heat absorption plate 731 into the container 30 for refrigerating the cold water, and the first heat absorption plate 731 is fixed to an inner periphery of the opening 32 of the cover 31, while sealing the container 30 and the opening 32 to prevent the cold water contained in the container 30 from leaking or flowing to the outside.

The heat dissipation fins 74 have a second heat absorption plate 741 stacked with the heat dissipation surface 72 and a plurality of second fins 742 extending from the second heat absorption plate 741, and the plurality of second fins 742 and the plurality of first fins 732 of the temperature conduction fins 73 extend in opposite directions, so that the waste heat generated by the operation of the cooling chip 70 can be dissipated through the second heat absorption plate 741 and the plurality of second fins 742.

In FIGS. 3 to 5, the compress strip 2 is formed by stacking two soft substrates 80 with each other by high-temperature welding, and a water path 81 is formed between two substrates 80 for circulating the cold water, and the outer surface of one of the substrates 80 has a heating film 82 separated from the water path 81 and configured in a staggered manner.

In an embodiment, the two substrates 80 of the compress strip 2 are a soft plastic film or a thin film respectively, and the water path 81 is bent between the two substrates 80, and the heating film 82 is made of a flexible material, and in a shape corresponding the gap of the water path 81 and arranged in a staggered manner.

The water path 81 has a water inlet 811 and a water outlet 812, and a tube 83 is installed between the water inlet 811 and water outlet 812 and the water pump 40 and passed between the upper and lower casings 10, 20 to communicate with the container 30.

The control unit 60 of the host machine 1 is electrically coupled to the water pump 40, the water valve 50 and the cooling chip 70, and electrically coupled to the heating film 82 of the compress strip 2 through a power cable 84 for controlling the ON/OFF of the water valve 50 and the tube 83, so that the cold water contained in the container 30 can be pumped from the tube 83 and the water inlet 811 through the water pump 40 and the water valve 50 into the water path 81 of the compress strip 2 and then flow from the water outlet 812 and the tube 83 back into the container 30 to define a water cycle, or the cold water in the water path 81 of the compress strip 2 is pumped back into the container 30.

When a user attaches the compress strip 2 onto an affected part on a human body surface, the cooling surface 71 of the cooling chip 70 absorbs the heat of the cold water contained in the container 30 through the plurality of temperature conduction fins 73, such that after the cold water is cooled and refrigerated by the cooling chip 70 and the temperature conduction fin 73, the cooled cold water is pumped by the water pump 40 of the host machine 1 to the water path 81 in the compress strip 2 through the plurality of tubes 83 for a circulation, so that the compress strip 2 attached on the affected part on the human body surface can perform a cold compress. In an embodiment, if a user believes that the water temperature is too low, the user can turn on the heating film 82 during the circulation of the cold water to adjust the water temperature to a comfortable temperature for the user.

If the user wants to perform a hot compress, the host machine 1 can quickly empty and pump the cold water in the water path 81 of the compress strip 2 by the water pump 40 and the water valve 50 back into the container 30 to prevent the cold water from flowing back into the compress strip 2, and then the control unit 60 controls the heating film 82 to generate heat, so that the compress strip 2 attached onto the affected part on the human body surface can perform a hot compress. In an embodiment, the control unit 60 controls different voltages applied to the heating film 82 to generate the heat with different temperatures and allow the user to adjust the hot compress temperature according to the user's requirement.

In an embodiment, in order to maintain the balance of pressure in the sealed container 30 to circulate the cold water smoothly, the cover 31 has an air hole 11 communicating to the interior of the container 30, so that when the host machine 1 empties and pumps the cold water contained in the compress strip 2 back into the container 30 by the water pump 40 and the water valve 50, the air hole 11 can be used to discharge the air in the container 30. When the water pump 40 of the host machine 1 starts pumping the cooled cold water into the compress strip 2 for circulation, the air hole 11 is provided for the air intake to maintain the balance of pressure in the container 30.

With the aforementioned structure of the present invention, the cooling source and the heating source are separated, so that when the cold/hot compress strip device performs a cold compress, the components such as the cooling chip 70 and the water pump 40 can be used to circulate and pump the cooled cold water into the compress strip 2 to perform the cold compress. For a hot compress, the cold water contained in the compress strip 2 is pumped and discharged, and then heated by the heating film 82 to perform the hot compress. The cold water in the water path 81 of the compress strip 2 is avoided from being heated, and the compress strip 2 can achieve an efficient cold/hot switch in an economic and energy-saving manner without the need of repeatedly heating and cooling the water as the prior art. In the aforementioned embodiment, if the compress strip 2 is used in an environment with a lower temperature such as room temperature lower than the water temperature for the cold compress, the heating film 82 can also be enabled to increase the cold water temperature during the circulation of the cold water.

In addition, the components in the host machine of the present invention reasonable and compact, and the conventional cooling chip, temperature conduction fin and heat dissipation fin can be used to achieve the functions of conducing cold temperature and dissipating heat as well as the effect of saving the manufacturing cost effectively.

In addition, the host machine 1 and the compress strip 2 can be connected or removed directly by the plurality of tubes 83 and power cable 84, or by a connection module 90 to allow the user to quickly replace the compress strip 2 according to the using requirement for the affected part of the human body surface.

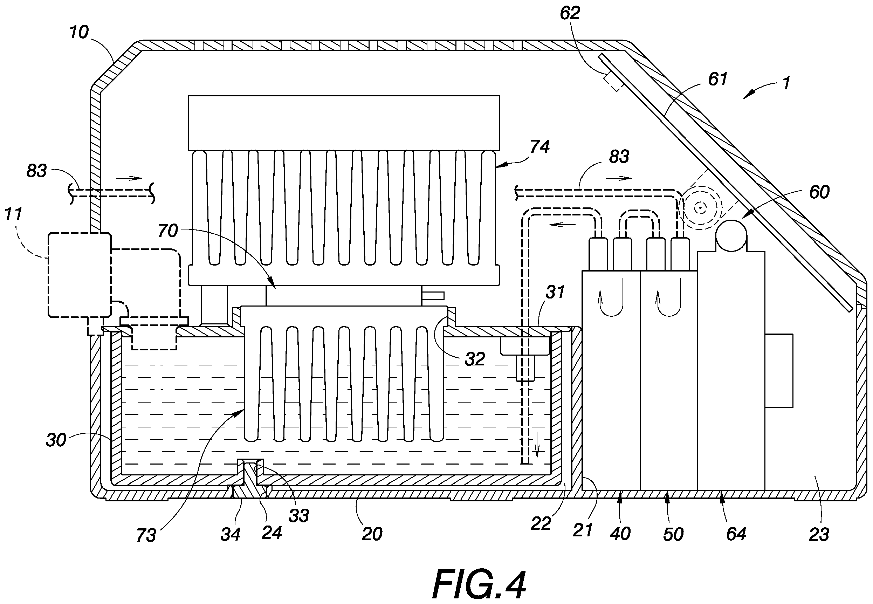

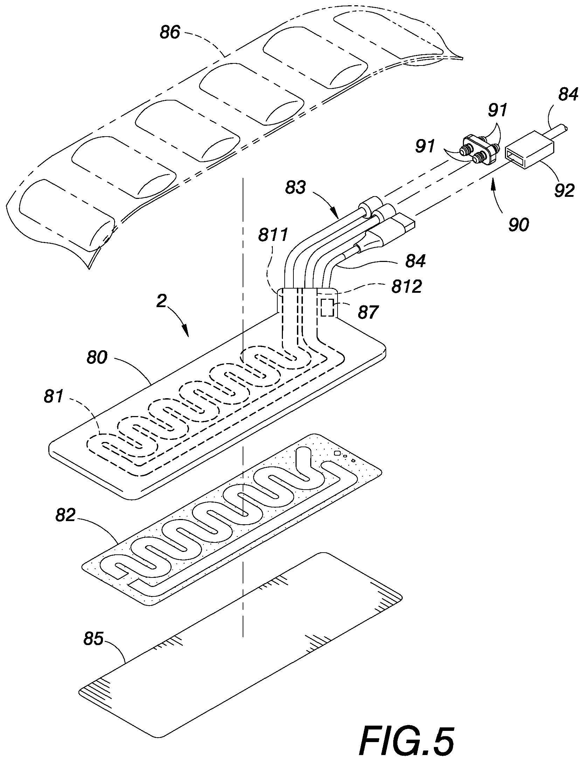

In FIGS. 4 and 5, the connection module 90 includes a plurality of sockets 91, and an electrical connector 92, the plurality of sockets 91 can be used for socketing the plurality of tubes 83 quickly to communicate the host machine 1 with the compress strip 2. The electrical connector 92 can be used for electrically connecting a power cable 84 between the host machine 1 and the compress strip 2, such that the host machine 1 can supply the electric power required for the operation of the compress strip 2, and the connection module 90 can be plugged or connected conveniently and easily to replace different sized compress strips 2, which are applicable for different affected parts of the human body. In an embodiment, the electrical connector 92 can be USB socket.

Other embodiments of the major components of the host machine 1 and the compress strip 2 are described below:

In an embodiment as shown in FIGS. 2 and 3, the bottom of the container 30 has a water injection port 33 communicating with the first accommodation space 22, and the bottom of the lower casing 20 has an external through hole 24 correspondingly communicating with the water injection port 33, and a plug 34 is detachably installed to the exterior of the through hole 24 for sealing the water injection port 33.

When the user carries the host machine 1 to the outside, the user can carry an empty container 30 in order to reduce the total weight, and the host machine 1 has a handle 12 pivotally installed to the exterior of the upper casing 10 to facilitate the user to carry the host machine 1.

When the user wants to use the compress device, the user can easily obtain room-temperature water from outside, or inject cold water or ice water into the container 30 from the water injection port 33, and then the cooling chip 70 and temperature conduction fins 73 can absorb the heat from the room-temperature water or cold water for refrigeration, and the container 30 can be designed and made of a thermal insulation material to prevent the water temperature from being disturbed by the ambient temperature, and can also assist achieving the effects of the cold water and saving energy.

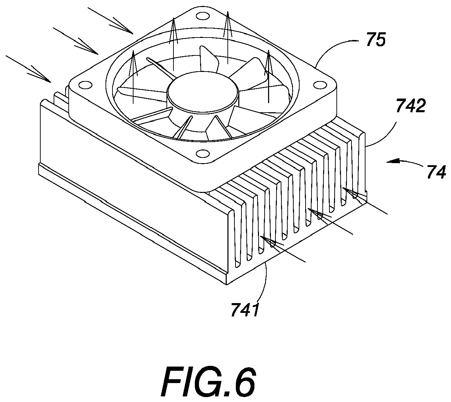

In an embodiment as shown in FIGS. 2 and 6, the upper casing 10 of the host machine 1 has a fan 75 capable of blowing and cooling the plurality of heat dissipation fins 74, and the upper casing 10 has a plurality of heat dissipating holes 13 formed around the periphery and the top of the fan 75, such that the waste heat generated by the operation of the cooling chip 70 can be dissipated into the environment through the heat dissipation fins 74 and the fan 75.

In FIGS. 1 to 3, the control unit 60 of an embodiment includes a circuit board 61, a wireless transmission module 62 capable of transmitting signal to an external mobile device, an operating interface 63 installed on a surface of the upper casing 10 for operating the cold/hot temperature, and a storage battery 64 for supplying electric power required for the operation of the host machine 1 and the compress strip 2.

By the control unit 60 of the aforementioned embodiment, the user can directly use the operating interface 63 to control the refrigerating/heating time and temperature and also can use the wireless transmission module 62 to connect the external mobile device via signal transmission, and the external mobile device can be used to set the refrigerating/heating time and temperature.

In an embodiment, the circuit board 61 can be installed at a position away from the plurality of heat dissipation fins 74 to ensure that sufficient air can flow between the upper and lower casings 10, 20 and to avoid the waste heat generated from the operation of the cooling chip 70 from being affected by the operation of the circuit board 61.

In FIG. 5, the exterior of one of the substrates 80 of the compress strip 2 further has a thermoconductive layer 85, and the thermoconductive layer 85 is laid in the water path 81 and on an outer surface of the heating film 82 and attached on a human body, which is capable of uniformly conducting the cold water in the water path 81 or the heat of the heating film 82 to the affected part of the human body. In an embodiment, the thermoconductive layer 85 is made of a material such as a flexible graphite sheet, or a flexible hydrogel sheet with a high thermal conductivity.

In addition, the exterior of the other substrate 80 of the compress strip 2 has a fastener 86 such as a hook and loop tape, a buckle strap or a buckle strap with a plurality of pressurized air bags disposed on the side opposite to the other side of the thermoconductive layer 85 for tying and fixing the compress strip 2 to the affected part of human body. When use, the compress strip 2 can be tied and fixed to the user's affected part, wherein the buckle strap with the plurality of pressurized air bags has an elastic compression effect to attach the aforementioned thermoconductive layer 85 more closely to the affected part to enhance the cold/hot compress effect.

In addition, the compress strip 2 of an embodiment further includes a temperature sensing module 87 capable of sensing a temperature, and the temperature sensing module 87 can sense a temperature value to assure that the compress strip 2 has achieve the predetermined cold/hot temperature. It is noteworthy that the temperature sensing module 87 can also be installed on the host machine.

While the invention has been described by means of specific embodiments, numerous modifications and variations could be made thereto by those skilled in the art without departing from the scope and spirit of the invention as set forth in the claims.

## Claims

1. A cold/hot compress strip device with a quick replaceable compress strip and an efficient cold/hot switch, comprising a host machine (1) and a flexible compress strip (2) detachably coupled to the host machine (1) and attachable to a human body surface, wherein the host machine (1) comprises a set of upper and lower casings (10, 20) vertically engaged with each other, and the lower casing (20) has a partition plate (21) disposed therein for dividing the lower casing (20) into first and second accommodation spaces (22,23), and the first accommodation space (22) contains a container (30) for containing cold water, and the top of the container (30) has a cover (31) for closing the container (30), and the cover (31) has an opening (32) formed thereon; the second accommodation space (23) contains a water pump (40), a water valve (50), and a control unit (60), for pumping and circulating the cold water; the upper casing (10) has a cooling chip (70) installed at a position above the opening (32) of the cover (31) and the cooling chip (70) has a cooling surface (71) and a heat dissipation surface (72), and the cooling surface (71) is stacked with a plurality of temperature conduction fins (73) fixed to an inner periphery of the opening (32) and extending into the container (30), and the heat dissipation surface (72) of the cooling chip (73) is stacked with a plurality of heat dissipation fins (74) fixed to the temperature conduction fin (73) and extending in an opposite direction away from the container (30); the compress strip (2) is formed by stacking two soft substrates (80) with each other, and a water path (81) between the two substrates (80) is provided for circulating the cold water, and a plurality of tubes (83) disposed between the water path (81) and the water pump (40) of the host machine (1) and installed between the upper and lower casings (10,20) are communicated with each other, and an outer surface of one of the substrates (80) has a heating film (82) separated from the water path (81); and the control unit (60) of the host machine (1) is electrically coupled to the water pump (40), the water valve (50) and the cooling chip (70), and electrically coupled to the heating film (82) of the compress strip (2) through a power cable for controlling the ON/OFF of the water valve (50) and the tubes (83), so that the water pump (40) can pump the cold water contained in the container (30) and cooled by the cooling chip (70) from the plurality of tubes (83) to the water path (81) in the compress strip (2) for circulation to perform a cold compress; or empty the cold water contained in the compress strip (2) back into the container (30), and control the heating film (82) to generate heat to perform a hot compress, so as to achieve an efficient cold/hot switch effect.

2. The cold/hot compress strip device with a quick replaceable compress strip and an efficient cold/hot switch according to claim 1, wherein the two substrates (80) of the compress strip (2) are a soft plastic film or a thin film respectively and stacked and welded to each other by a high-temperature welding technology, and the water path (81) is bent between the two substrates (80) and the water path (81) has a water inlet (811) and a water outlet (812) communicating with the plurality of tubes (83) respectively, and the heating film (82) is in a shape corresponding to that of a gap of the water path (81) and arranged in a staggered manner.

3. The cold/hot compress strip device with a quick replaceable compress strip and an efficient cold/hot switch according to claim 2, wherein one of the substrates (80) of the compress strip (2) further has a thermoconductive layer (85) disposed on the outside thereof and laid on an the water path (81) and an outer surface of the heating film (82), and the thermoconductive layer (85) is capable of uniformly conducting the cold/hot temperature generated by the water path (81) or the heating film (82) to an affected part of a human body.

4. The cold/hot compress strip device with a quick replaceable compress strip and an efficient cold/hot switch according to claim 3, wherein the other substrate (80) of the compress strip (2) further has a hook and look tape or a buckle strap disposed on the side opposite to the other side of the thermoconductive layer (85) respectively and capable of tying and fixing the compress strip (2) to the affected part of the human body, or has a buckle strap with a plurality of pressurized air bags.

5. The cold/hot compress strip device with a quick replaceable compress strip and an efficient cold/hot switch according to claim 3, wherein the compress strip (2) further has a temperature sensing module (87) installed thereon and capable of sensing a temperature.

6. The cold/hot compress strip device with a quick replaceable compress strip and an efficient cold/hot switch according to claim 1, wherein the host machine (1) and the compress strip (2) has a connection module (90) installed therebetween and capable of quickly install or remove the compress strip (2), and the connection module (90) comprises a plurality of sockets (91) for socketing the plurality of tubes (83) to communicate the host machine (1) with the compress strip (2), and an electrical connector (92) provided for electrically connecting a power cable between the host machine (1) and the compress strip (2).

7. The cold/hot compress strip device with a quick replaceable compress strip and an efficient cold/hot switch according to claim 1, wherein the container (30) has a water injection port (33) formed at the bottom thereof and communicating with the first accommodation space (22), and the bottom of the lower casing (20) has an external communicating through hole (24) configured to be corresponsive to the water injection port (33), and a plug (34) detachably installed to an external part of the through hole (24) and capable of sealing the water injection port (33).

8. The cold/hot compress strip device with a quick replaceable compress strip and an efficient cold/hot switch according to claim 1, wherein the upper casing (10) has a fan (75) installed therein and capable of blowing and cooling the heat dissipation fin (74), and a plurality of heat dissipating holes (13) formed around and above the fan (75).

9. The cold/hot compress strip device with a quick replaceable compress strip and an efficient cold/hot switch according to claim 8, wherein the control unit (60) comprises a circuit board (61), an operating interface (63) installed on a surface of the upper casing (10) and provided for operating the cold/hot temperature, and a storage battery (64) for supplying an electric power required for the operation of the host machine (1) and the compress strip (2).

10. The cold/hot compress strip device with a quick replaceable compress strip and an efficient cold/hot switch according to claim 9, wherein the control unit (60) further comprises a wireless transmission module (62) for a signal transmission with an external mobile device.
